(19) Europäisches Patentamt / European Patent Office / Office européen des brevets

(11) **EP 4 353 600 A1**

(12) **EUROPEAN PATENT APPLICATION**

(43) Date of publication:
**17.04.2024  Bulletin 2024/16**

(21) Application number: **23203861.2**

(22) Date of filing: **16.10.2023**

(51) International Patent Classification (IPC):
**B64D 47/02** (2006.01)   **A61L 2/10** (2006.01)
**B64D 11/00** (2006.01)   **G06F 3/01** (2006.01)

(52) Cooperative Patent Classification (CPC):
**B64D 47/02; A61L 2/10; G06F 3/01; B60Q 3/68;**
**B64D 2011/0038**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **14.10.2022  US 202217966561**

(71) Applicant: **B/E Aerospace, Inc.**
**Winston-Salem, NC 27105 (US)**

(72) Inventors:
• **WEICHSELBAUM, Noah Avram Meltz**
**Prarie Village, KS, 66208 (US)**
• **EDQUIST, John D.**
**Milwaukee, WI, 53207 (US)**
• **JOHANNESSEN, Eric**
**Holbrook, NY, 11741 (US)**

(74) Representative: **Dehns**
**St. Bride's House**
**10 Salisbury Square**
**London EC4Y 8JD (GB)**

(54) **EMBEDDED NEAR-ULTRAVIOLET SELF-CLEANING ELECTRONICS FOR PASSENGER CONTROL UNIT (PCU)**

(57)     A self-disinfecting passenger control unit (PCU) includes a transparent or translucent molded or injected polycarbonate layer (300) fused between inner and outer films, the outer film divided into contact surfaces (e.g., for passenger control of lights, call buttons, seats, and other functions) and the inner film including embedded traces. Within the molded polycarbonate layer are embedded LED units (108) configured for continuous near-ultraviolet (near-UV) emission (e.g., at or near 405 nm), providing a uniform luminous output directed at the outer film and capable of antiviral/antibacterial irradiation of the high-touch contact surfaces of the outer film. As the continuous near-UV output is both visible by, and safe for, passengers, the LED units may double as back-lighting for guiding passengers to the correct contact surface for adjusting a desired cabin function.

EP 4 353 600 A1

*FIG. 3*

**EP 4 353 600 A1**

**Description**

BACKGROUND

Passenger control units (PCU; also, passenger service units (PSU)) enable aircraft passengers to adjust the configuration of their passenger seat, adjust the surrounding environment, and interact with the flight crew and/or the aircraft in other ways. As such, PCUs are generally high touch surfaces. For example, a passenger may make repeated physical contact with a PCU during the course of a flight. If a given aircraft arrives, via a first flight, at an airport with an initial passenger complement and later departs said airport via a second flight with a partially or fully new passenger complement, each PCU (in addition to other high touch surfaces in galley areas, lavatory areas, and elsewhere throughout the passenger cabin) is a vehicle for potential transmission of influenza-A, COVID-19, and other communicable microorganisms.

PCU modules may be illuminated to provide a passenger with visual guidance that facilitates PCU operation in a darkened cabin. Ultraviolet light (UV), or luminous output of certain wavelengths within the ultraviolet spectrum (e.g., 10-400 nm), has been shown effective at disinfecting high touch surfaces via prolonged exposure. However, challenges are associated with conventional approaches to disinfecting high touch surfaces via UV. For example, ultraviolet-C wavelengths (UVC or UV-C; e.g., 254 nm, 275 nm, or wavelengths 280 nm or less generally) that are most effective with respect to exposure time are potentially harmful to human tissue. However, the longer exposure time (e.g., > 1 hr) required for ultraviolet-A (UVA or UV-A; e.g., 315 nm or greater) to be human safe may be a prohibitively long time for an aircraft to remain grounded and empty while high touch surfaces are disinfected. Similarly, UVC wavelengths at 222 nm are safe for humans due to the shorter wavelengths, but require cumbersome and expensive noble gas-filled excimer lamps for UV emission.

SUMMARY

In an aspect, a self-cleaning passenger control unit (PCU) for an aircraft cabin is disclosed. In embodiments, the self-cleaning PCU includes an embedded electronic component installable in a cabin surface (for example, a set of contact surfaces allowing a passenger to control seat configuration, reading lights, cabin crew call, ventilation, in-flight entertainment (IFE) and/or other cabin services or functions. The embedded electronic component includes inner and outer films fused or bonded to inner and outer faces of an injected or molded transparent polycarbonate layer. The contact surfaces are set into the outer film and therefore are high-touch surfaces associated with frequent and/or long-term passenger contact. Each contact surface may be labeled to identify the cabin surface controllable by that contact surface. The inner film includes embedded electronic contacts (e.g., circuitry for receiving control input through the contact surfaces and transmitting control signals to the appropriate cabin services) and embedded near-ultraviolet (near-UV) LED units configured for continuous luminous output in the near-UIV range (e.g., wavelengths at or near 405 nm). The near-UV luminous output is directed through the injected polycarbonate layer to irradiate and disinfect the outer high-touch contact surfaces. The PCU includes an interface pluggable into aircraft systems and networks, allowing for transmission of control signals from the PCU to the appropriate cabin system or function.

In some embodiments, the near-UV LED units are oriented for lateral or indirect emission relative to the outer film and contact surfaces. For example, the injected polycarbonate layer provides waveguides for directing the near-UV luminous output to the outer film.

In some embodiments, the inner film includes printed patterns for directing and/or diffusing the near-UV luminous output.

In some embodiments, the PCU includes negative contact surfaces, e.g., an illuminated transparent or translucent background and opaque or semi-opaque graphic elements identifying the controlled cabin system.

In some embodiments, the PCU includes positive contact surfaces, e.g., an opaque or semi-opaque background and illuminated graphic elements.

In some embodiments, the PCU incorporates three-dimensional (3D) graphic elements extending above the surrounding contact surface.

In some embodiments, the controllable cabin systems include seat configurations, reading or cabin lights, fans or vents, or cabin crew call signals.

In some embodiments, the PCU includes additional LED units for altering the color of the luminous output, e.g., if the blue color of the near-UV LED output is undesirable.

In some embodiments, the PCU is a multiplanar PCU incorporating contact surfaces in multiple or staggered planes.

In some embodiments, the PCU is a partially or fully nonplanar PCU incorporating curved contact surfaces and/or conforming/contouring to a curved/nonplanar cabin surface. This Summary is provided solely as an introduction to subject matter that is fully described in the Detailed Description and Drawings. The Summary should not be considered to describe essential features nor be used to determine the scope of the Claims. Moreover, it is to be understood that both the foregoing Summary and the following Detailed Description are example and explanatory only and are not necessarily restrictive of the subject matter claimed.

BRIEF DESCRIPTION OF THE DRAWINGS

The detailed description is described with reference to the accompanying figures. The use of the same reference numbers in different instances in the description and the figures may indicate similar or identical items. Various embodiments or examples ("examples") of the present disclosure are disclosed in the following detailed description and the accompanying drawings. The drawings are not necessarily to scale. In general, operations of disclosed processes may be performed in an arbitrary order, unless otherwise provided in the claims. In the drawings:

FIG. 1 is an isometric view illustrating a self-cleaning/self-disinfecting passenger control unit (PCU) according to with example embodiments of this disclosure;

FIG. 2 is an interior view of the self-cleaning PCU of FIG. 1;

FIG. 3 is an exploded view of an embedded electronic component of the self-cleaning PCU of FIG. 1;

FIG. 4 is a profile view of the self-cleaning PCU of FIG. 1;

FIG. 5 is a detailed view of the self-cleaning PCU of FIG. 1, showing additional LED units embedded therein according to example embodiments of this disclosure;

FIG. 6 is a detailed view of the self-cleaning PCU of FIG. 1, showing three-dimensional graphical elements according to example embodiments of this disclosure;

FIG. 7 is an isometric view of a multiplanar self-cleaning PCU according to example embodiments of this disclosure; and

FIG. 8 is an isometric view of a nonplanar self-cleaning PCU according to example embodiments of this disclosure.

DETAILED DESCRIPTION

Before explaining one or more embodiments of the disclosure in detail, it is to be understood that the embodiments are not limited in their application to the details of construction and the arrangement of the components or steps or methodologies set forth in the following description or illustrated in the drawings. In the following detailed description of embodiments, numerous specific details may be set forth in order to provide a more thorough understanding of the disclosure. However, it will be apparent to one of ordinary skill in the art having the benefit of the instant disclosure that the embodiments disclosed herein may be practiced without some of these specific details. In other instances, well-known features may not be described in detail to avoid unnecessarily complicating the instant disclosure.

As used herein a letter following a reference numeral is intended to reference an embodiment of the feature or element that may be similar, but not necessarily identical, to a previously described element or feature bearing the same reference numeral (e.g., 1, 1a, 1b). Such shorthand notations are used for purposes of convenience only and should not be construed to limit the disclosure in any way unless expressly stated to the contrary.

Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of "a" or "an" may be employed to describe elements and components of embodiments disclosed herein. This is done merely for convenience and "a" and "an" are intended to include "one" or "at least one," and the singular also includes the plural unless it is obvious that it is meant otherwise.

Finally, as used herein any reference to "one embodiment" or "some embodiments" means that a particular element, feature, structure, or characteristic described in connection with the embodiment is included in at least one embodiment disclosed herein. The appearances of the phrase "in some embodiments" in various places in the specification are not necessarily all referring to the same embodiment, and embodiments may include one or more of the features expressly described or inherently present herein, or any combination or sub-combination of two or more such features, along with any other features which may not necessarily be expressly described or inherently present in the instant disclosure.

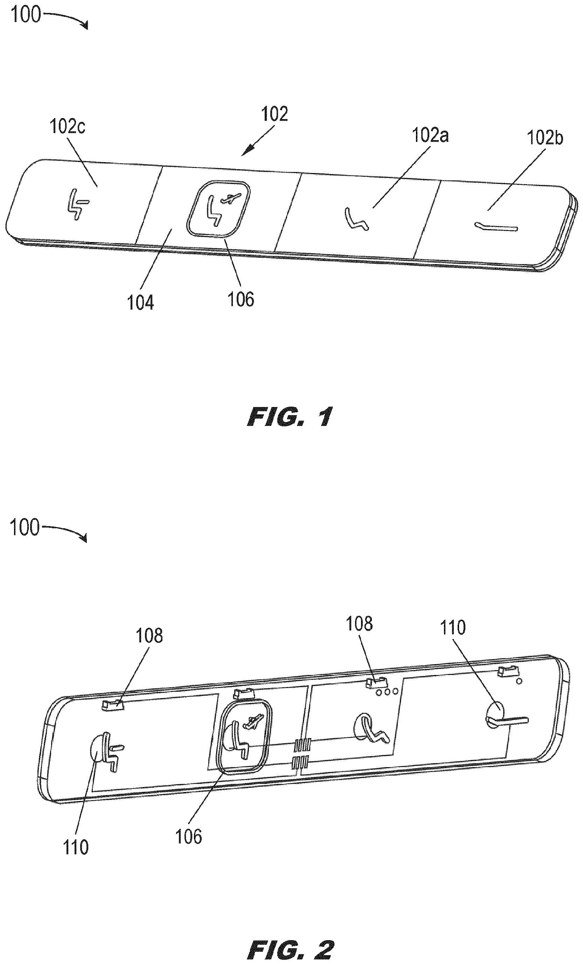

Referring to FIG. 1, a self-cleaning/self-disinfecting PCU 100 is shown.

In embodiments, the PCU 100 may be disposed on or near a passenger seat, e.g., either incorporated into the passenger seat or set into a wall surface or other surface proximate to the passenger seat. For example, the PCU 100 may be incorporated into or near a particular passenger seat for use by the passenger occupying said passenger seat. In embodiments, the exterior surface of the PCU 100 may be divided into a set of individual contact surfaces 102 (e.g., button surfaces), each contact surface dedicated to the control of a particular cabin system or cabin function by the occupying passenger. For example, individual contact surfaces 102 may allow the passenger, via physical contact with the contact surface, to, e.g.: call or alert a crewmember; activate/deactivate a reading light; convert the passenger seat to a reclined (102a) or berthed (102b) configuration; return the passenger seat to the upright configuration (102c); activate/deactivate a fan or gasper; engage with an in-flight entertainment (IFE) system; and/or interact with the aircraft and/or flight crew in various other ways. It may be noted that the individual contact surfaces 102, 102a-102c are high-touch surfaces in that one or more contact surfaces may be subject to frequent physical contact, e.g., either by a single passenger or by multiple passengers and/or crewmembers.

In embodiments, referring also to FIG. 2, each individual contact surface 102, 102a-c may be illuminated from within, such that each contact surface is sanitized via continuous near-ultraviolet (near-UV) irradiation. For example, the self-cleaning PCU 100 may incorporate embedded electronic components which provide continuous irradiation of the contact surfaces 102 to luminous output in the near-ultraviolet (near UV) range, disinfecting the contact surfaces of any viral and bacterial microorganisms which may be deposited there via repeated passenger contact.

For example, each contact surface 102, 102a-c may be a negative contact surface incorporating transparent or translucent background layer 104 and one or more opaque or semi-opaque graphic elements 106. In embodiments, the background layer 104 may be fully transparent or translucent, such that the background layer is backlit via one or more light emitting diode (LED) units 108 embedded within the PCU 100. For example, the background layer 104 may be backlit to facilitate distinction of one contact surface 102, 102a-c from the other contact surfaces, e.g., for ease of use of the PCU 100 in a darkened cabin, and for near-UV disinfection of the contact surfaces via the embedded LED units 108.

In embodiments, the PCU 100 may include electronic contacts 110 embedded within the PCU. For example, each electronic contact 110 may include circuitry for carrying control input provided by a passenger or other user (e.g., via engagement with a contact surface 102, 102a-102c) to the device or cabin system controlled by said contact surface, as discussed in detail below.

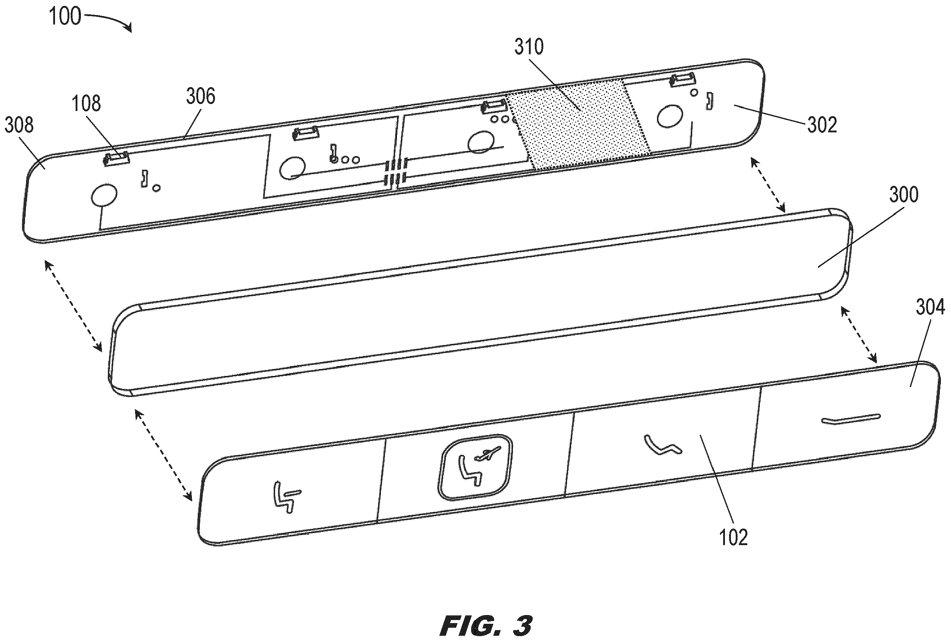

Referring now to FIG. 3, the self-cleaning PCU 100 is shown.

In embodiments, the PCU 100 may comprise an embedded electronic component wherein an injected polycarbonate layer 300 (e.g., molded layer) is fused between an outer film 304 (e.g., exterior substrate) and an inner film 302 (e.g., interior substrate). For example, the high-touch contact surfaces 102 may be incorporated into the outer film 304, such that the outer film may be divided into a set of contact surfaces, each contact surface including one or more graphic elements 106. Similarly, the LED units 108 and electronic contacts 110 may be attached to the inner film 302, the LED units attached near an edge 306 of the PCU 100 and the electronic contacts centered behind each contact surface 102, such that the LED units and electronic traces are embedded within the injected polycarbonate layer 300. In some embodiments, the LED units 108 may be attached to the inner film 302 near an intersection of edges 308 or otherwise not directly centered under a transparent or translucent portion of the outer film 304 and/or contact surfaces 102.

In embodiments, the near-UV luminous output of the LED units 108 may include wavelengths between 380 nm and 420 nm, preferably at or around 405 nm in wavelength. For example, the near-UV luminous output of the LED units 108 may be directed at and through the outer film 304 from the inside, continuously irradiating the outer film and its component contact surfaces 102 and providing long-term disinfection of the contact surfaces against microorganisms deposited there by passenger contact surface. In embodiments, the LED units 108 may be configured for lateral (e.g., indirect) luminous emission; for example, the luminous output of the LED units may not be aimed directly at the contact surfaces 102 or the outer film 304, but may be directed thereto via the injected polycarbonate layer 300.

Prolonged exposure to near-UV luminous emissions at or around 405 nm have been shown effective against influenza-A, COVID-19, Staphylococcus aureus, and other viral/bacterial microorganisms. In embodiments, the LED units 108 may provide continuous self-disinfection of the contact surfaces 102 through continuous exposure to near-UV luminous output. For example, unlike other wavelengths in the UVC spectrum with disinfectant properties, luminous output at 405 nm is not associated with long-term damage to human tissue. Accordingly, the continuous luminous output at 405 nm presents no danger to passengers in the vicinity of the luminous output, even if the LED units 108 remain active while the passenger cabin is occupied, e.g., inflight. In embodiments, the near-UV luminous output of the LED units 108 may be associated with a soft blue tone that may be acceptable for use as backlighting to the contact surfaces 102 as described above.

In some embodiments, the embedded electronic component may include diffusive patterns 310 printed onto the inner film 302. For example, the diffusive patterns 310 may promote the direction and/or diffusion of the near-UV luminous output of the LED units 108, such that the luminous output of the LED units (e.g., and the irradiation of the contact surfaces 102) is uniform as well as continuous.

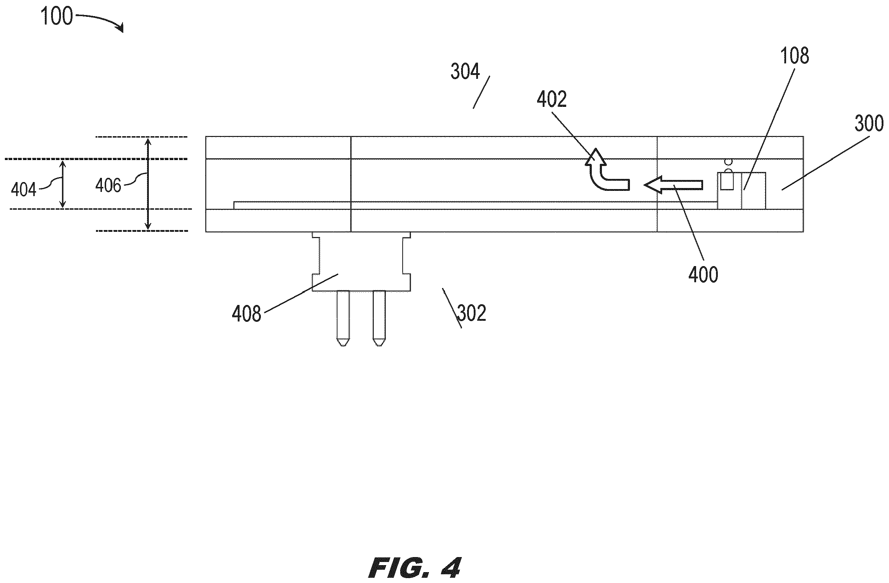

Referring now to FIG. 4, the self-cleaning PCU 100 is shown.

In embodiments, the efficacy of the self-cleaning PCU 100 in disinfecting the contact surfaces (102, FIG. 3) may depend on several factors including the ratio of near-UV LED units 108 to the surface area of the outer film 304 and the transmittance of the injected polycarbonate layer 300. For example, the dosage of 405 nm near-UV radiation received by the contact surfaces 102 (e.g., in joules per cm$^2$) may be function of irradiance, e.g.:

$$dosage \left(\frac{J}{cm^2}\right) = irradiance \left(\frac{mW}{cm^2}\right) * transmittance * time \ (sec) \qquad [1]$$

where

$$irradiance \left(\frac{mW}{cm^2}\right) = \frac{LED \ luminous \ flux \ (lm)*(number \ of \ LED \ units)}{PCU \ surface \ area}. \qquad [2]$$

In embodiments, the irradiance shown by equation 2 may be optimized by the incorporation by the PCU 100 of additional near-UV LED units 108. For example, the PCU 100 may be configured for higher or lower irradiance based on, e.g., the placement of the PCU relative to a passenger cabin or to a passenger (PCUs associated with passenger seats may be configured for lower overall irradiance while PCUs in higher traffic illuminated areas (or, e.g., handles, switches, and other high touch surfaces in lavatory or galley areas) may be configured for higher overall irradiance).

In embodiments, the PCU 100 may improve upon conventional backlit embedded electronics by eliminating an air gap between the outer and inner films 302, 304. For example, the substantially transparent or translucent injected polycarbonate layer 300 may be fused between outer and inner films 302, 304 such that the polycarbonate layer extends fully between the outer and inner films and may serve as a waveguide for directing the lateral luminous output 400 of the LED units 108 toward (402) the outer film and its component contact surfaces (102, FIG. 3) in a uniform fashion.

In embodiments, by embedding the LED units 108 within to the inner film 302 and fusing the outer film 304 and inner film to opposing faces of the injected polycarbonate layer 300, the distance between the near-UV LED units 108 and the outer film (and its component contact surfaces 102) may be significantly reduced with respect to a conventional backlit PCU. For example, while the conventional backlit PCU may incorporate a 4 mm internal polycarbonate layer (plus a significant air gap) between the inner electronics and the outer surface, the embedded electronic component of the self-cleaning PCU 100 may incorporate a significantly thinner injected polycarbonate layer 300 (e.g., a width 404 of 2 mm or less) and eliminate the air gap entirely (e.g., a total width 406 of 4 mm for the embedded electronic component as a whole). In embodiments, the reduction in distance between the near-UV LED units 108 and the outer film 304 may be associated with a 20 percent increase in transmittance via the injected polycarbonate layer 300 (e.g., based on the 4 mm - 2 mm reduction in width described above).

In embodiments, the PCU 100 may include an interface 408 insertable into a port (not shown) set within the cabin surface into which the PCU is installed. For example, the port may provide (e.g., via physical power and/or data connections) links to cabin systems and aircraft networks such that when the PCU 100 is installed, control input provided via the contact surfaces 102 by a user may be carried as control signals via the electronic contacts (110, FIG. 1) and through the interface 402 to the corresponding cabin systems.

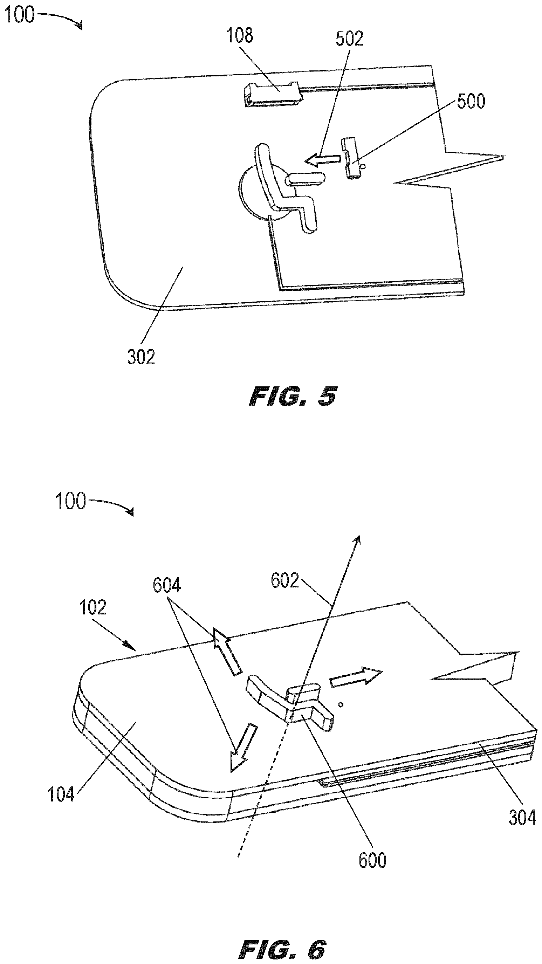

Referring now to FIG. 5, the self-cleaning PCU 100 is shown. In some embodiments, the PCU 100 may incorporate additional LED units 500 embedded into the inner film 302 and the injected polycarbonate layer (300, FIG. 4). For example, the additional LED units 500 may provide continuous luminous output at other desired wavelengths within the visible spectrum (c. 380-700 nm) to correct the blue color otherwise associated with the near-UV LED units 108 (e.g., if said color is undesirable). Accordingly, the visible luminous output through the outer film (304, FIG. 4) may retain the disinfectant properties of the luminous output of the near-UV LED units 108, but may appear as light of another desired color, including white light, depending on the distribution and/or wavelengths of the additional LED units 500.

Referring now to FIG. 6, the self-cleaning PCU 100 is shown.

In some embodiments, the individual contact surfaces 102 of the PCU 100 may incorporate three-dimensional graphic elements 600. For example, the three-dimensional graphic elements 600 may be raised, or may extend above the background layer 104 of the contact surface relative to a z-axis 602.

In some embodiments, the PCU 100 may incorporate positive contact surfaces 102 (as opposed to the negative contact surfaces shown, e.g., by FIG. 1). rather than positive, contact surfaces 102. For example, a positive contact surface 102 may incorporate an opaque or semi-opaque background layer 106 and translucent or transparent graphic elements 108, 600, such that the contact surface 102 is backlit, and disinfected, via near-UV luminous output 604 directed from the near-UV LED units (108, FIG. 5) toward the outer film 304 and through the graphic elements (104, FIG. 1).

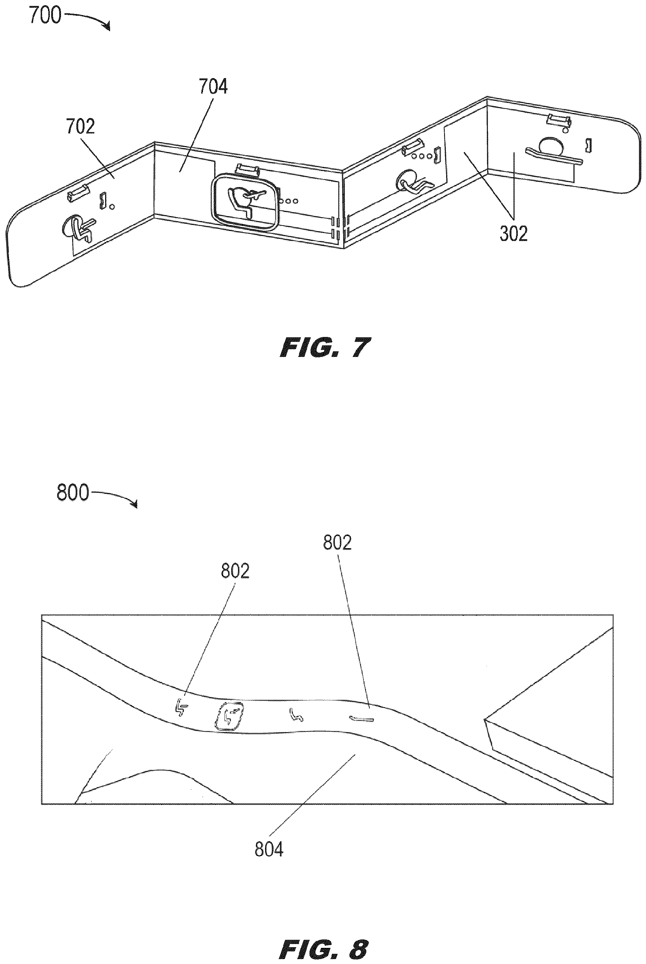

Referring to FIG. 7, the multiplanar self-cleaning PCU 700 may be implemented and may function similarly to the self-cleaning PCU 100 of FIGS. 1 through 6, except that the multiplanar PCU 700 may incorporate an inner film 302, outer film (304, FIG. 4), injected polycarbonate layer (300, FIG. 4) and contact surfaces 702, 704 in multiple and/or staggered planes. For example, the multiplanar self-cleaning PCU 700 may be fashioned to contour or conform to a multiplanar cabin surface.

Referring to FIG. 8, the nonplanar self-cleaning PCU 800 may be implemented and may function similarly to the self-cleaning PCU 100 of FIGS. 1 through 6, except that the inner film 302, outer film 304, injected polycarbonate layer 300, and contact surfaces 802 may be partially or fully nonplanar. For example, the nonplanar self-cleaning PCU 800 may be fashioned to conform or contour to a curved cabin surface 804, such that each contact surface 802 may be curved or nonplanar throughout its individual surface area, or that the set of contact surfaces collectively conforms to the curved cabin surface.

Embodiments of the inventive concepts disclosed herein may improve upon conventional PCUs by providing a safe and perpetual self-disinfecting capability that is "always on", e.g., provides continuous disinfection of high touch surfaces at relatively low power. The self-disinfecting PCU may provide continuous protection by remaining active inflight without potential harm to passengers or flight crew. In embodiments, recommended near-UV dosage for eliminating viral and bacterial microorganisms may be realized with 30 minutes or less of exposure.

CONCLUSION

It is to be understood that embodiments of the methods disclosed herein may include one or more of the steps described herein. Further, such steps may be carried out in any desired order and two or more of the steps may be carried out simultaneously with one another. Two or more of the steps disclosed herein may be combined in a single step, and in some embodiments, one or more of the steps may be carried out as two or more sub-steps. Further, other steps or sub-steps may be carried in addition to, or as substitutes to one or more of the steps disclosed herein.

Although inventive concepts have been described with reference to the embodiments illustrated in the attached drawing figures, changes may be made without departing from the scope of the claims. Components illustrated and described herein are merely examples of a system/device and components that may be used to implement embodiments of the inventive concepts and may be replaced with other devices and components without departing from the scope of the claims. Furthermore, any dimensions, degrees, and/or numerical ranges provided herein are to be understood as non-limiting examples unless otherwise specified in the claims.

**Claims**

1. A passenger control unit, PCU, comprising:
   an embedded electronic component installable within a cabin surface of an aircraft, the embedded electronic component comprising:

   at least one interface (408) configured for operatively coupling the PCU to a plurality of externally controlled cabin systems via at least one of a physical connection or an aircraft network;
   a translucent injected polycarbonate layer (300) fused between an exterior substrate and an interior substrate,

   the exterior substrate including a plurality of contact surfaces (102, 102a - c), each contact surface configured for providing control input to a cabin system of the plurality of cabin systems in response to physical contact by a user, each contact surface including at least one graphic element corresponding to the cabin system, the interior substrate including embedded circuitry configured for connecting each contact surface to the interface and for transmitting the control input to the corresponding cabin system via the interface;

   and
   one or more light emitting diode, LED, units (108) embedded within the interior substrate proximate to at least one edge of the embedded electronic component, the one or more LED units configured to direct a continuous luminous output of wavelengths within a range from 380 nm to 420 nm to the exterior substrate via the injected polycarbonate layer.

2. The PCU of Claim 1, wherein:

   the one or more LED units (108) are oriented for lateral emission relative to the exterior substrate;
   and

the translucent injected polycarbonate layer (300) is configured for directing the continuous luminous output toward the exterior substrate.

3. The PCU of Claim 1 or 2, further comprising:
   at least one pattern printed onto the interior substrate and configured for diffusion of the continuous luminous output.

4. The PCU of Claim 1, 2 or 3, wherein the plurality of contact surfaces includes at least one negative contact surface comprising:

   an icon portion associated with a first opacity and corresponding to the at least one graphic element; and
   a background portion associated with a second opacity lower than the first opacity.

5. The PCU of any preceding Claim, wherein the plurality of contact surfaces includes at least one positive contact surface comprising:

   an icon portion associated with a first opacity and corresponding to the at least one graphic element; and
   a background portion associated with a second opacity higher than the first opacity.

6. The PCU of Claim 5, wherein the icon portion extends above the exterior substrate relative to a z-axis.

7. The PCU of any preceding Claim, wherein the plurality of externally controlled cabin systems includes one or more of:

   a passenger seat adjustable by the user via the PCU;
   a light controllable by the user via the PCU;
   a ventilation unit controllable by the user via the PCU;
   or
   a call signal configured for alerting a crewmember of the aircraft.

8. The PCU of any preceding Claim, wherein the one or more LED units are first LED units and the range is a first range, further comprising:
   one or more second LED units embedded within the interior substrate, the one or more second LED units configured for colored luminous output associated with one or more wavelengths within a second range from 365 nm to 660 nm but excluding the first range, the colored luminous output associated with at least one desired color associated with the second range.

9. The PCU of any preceding Claim, wherein the embedded electronic component has a partially multiplanar configuration wherein the plurality of contact surfaces comprises:

   a first contact surface;
   and
   at least one second contact surface not coplanar with the first contact surface.

10. The PCU of any preceding Claim, wherein the embedded electronic component has an at least partially nonplanar configuration.

**FIG. 1**

**FIG. 2**

**FIG. 3**

EP 4 353 600 A1

**FIG. 4**

EP 4 353 600 A1

**FIG. 5**

**FIG. 6**

700

702  704

302

**FIG. 7**

800

802  802

804

**FIG. 8**

Europäisches
Patentamt

European
Patent Office

Office européen
des brevets

# EUROPEAN SEARCH REPORT

Application Number

EP 23 20 3861

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 10 667 396 B2 (TACTOTEK OY [FI]) 26 May 2020 (2020-05-26) | 1-7,9,10 | INV. B64D47/02 A61L2/10 B64D11/00 G06F3/01 |
| A | * claim 1 * <br> * figures 1-5 * <br> * column 14, line 37 - line 45 * <br> * column 15, line 14 - line 25 * | 8 | |
| Y | WO 2021/254619 A1 (HUAWEI TECH CO LTD [CN]; WIPIEJEWSKI TORSTEN [DE]) 23 December 2021 (2021-12-23) | 1-7,9,10 | |
| A | * claims 1, 5 * | 8 | |
| A | US 2022/088243 A1 (TAKAHATA MASASHI [JP]) 24 March 2022 (2022-03-24) * the whole document * | 1-10 | |
| A | US 2015/174276 A1 (TUMANOV ILYA [US]) 25 June 2015 (2015-06-25) * the whole document * | 1-10 | |
| A | US 10 377 351 B2 (FORD GLOBAL TECH LLC [US]) 13 August 2019 (2019-08-13) * the whole document * | 1-10 | TECHNICAL FIELDS SEARCHED (IPC) <br><br> B64D <br> A61L |
| A | US 2022/055751 A1 (HANSSON CHARLES MARTIN [US] ET AL) 24 February 2022 (2022-02-24) * the whole document * | 1-10 | |
| A | US 2022/288253 A1 (YAHNKE CLIFFORD J [US]) 15 September 2022 (2022-09-15) * the whole document * | 1-10 | |
| A | US 2021/379217 A1 (BECKMAN JOHN C [US]) 9 December 2021 (2021-12-09) * the whole document * | 1-10 | |

The present search report has been drawn up for all claims

| Place of search | Date of completion of the search | Examiner |
|---|---|---|
| Munich | 28 February 2024 | Duval, Yann |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 23 20 3861

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2024

| Patent document cited in search report | | Publication date | Patent family member(s) | | Publication date |
|---|---|---|---|---|---|
| US 10667396 | B2 | 26-05-2020 | CN | 111052885 A | 21-04-2020 |
| | | | EP | 3673719 A1 | 01-07-2020 |
| | | | JP | 2020532132 A | 05-11-2020 |
| | | | KR | 20200042913 A | 24-04-2020 |
| | | | TW | 201922066 A | 01-06-2019 |
| | | | US | 2019069408 A1 | 28-02-2019 |
| | | | US | 2019069409 A1 | 28-02-2019 |
| | | | WO | 2019038479 A1 | 28-02-2019 |
| WO 2021254619 | A1 | 23-12-2021 | CN | 115768488 A | 07-03-2023 |
| | | | WO | 2021254619 A1 | 23-12-2021 |
| US 2022088243 | A1 | 24-03-2022 | JP | 2022051407 A | 31-03-2022 |
| | | | US | 2022088243 A1 | 24-03-2022 |
| US 2015174276 | A1 | 25-06-2015 | US | 2013045132 A1 | 21-02-2013 |
| | | | US | 2015174276 A1 | 25-06-2015 |
| US 10377351 | B2 | 13-08-2019 | CN | 109131078 A | 04-01-2019 |
| | | | DE | 102018115394 A1 | 03-01-2019 |
| | | | GB | 2565896 A | 27-02-2019 |
| | | | US | 2019001933 A1 | 03-01-2019 |
| US 2022055751 | A1 | 24-02-2022 | EP | 3957564 A1 | 23-02-2022 |
| | | | US | 2022055751 A1 | 24-02-2022 |
| US 2022288253 | A1 | 15-09-2022 | AU | 2022235283 A1 | 14-09-2023 |
| | | | CA | 3209706 A1 | 15-09-2022 |
| | | | US | 2022288253 A1 | 15-09-2022 |
| | | | WO | 2022192679 A1 | 15-09-2022 |
| US 2021379217 | A1 | 09-12-2021 | CN | 113827744 A | 24-12-2021 |
| | | | EP | 3922273 A1 | 15-12-2021 |
| | | | JP | 2021192791 A | 23-12-2021 |
| | | | US | 2021379217 A1 | 09-12-2021 |

EPO FORM P0459

For more details about this annex : see Official Journal of the European Patent Office, No. 12/82